(19)

Europäisches
Patentamt
European
Patent Office
Office européen
des brevets

(11) **EP 4 371 468 A1**

(12) **EUROPEAN PATENT APPLICATION**

(43) Date of publication:
**22.05.2024  Bulletin 2024/21**

(21) Application number: **22306676.2**

(22) Date of filing: **15.11.2022**

(51) International Patent Classification (IPC):
**A61B 3/028** $^{(2006.01)}$     **A61B 3/032** $^{(2006.01)}$

(52) Cooperative Patent Classification (CPC):
**A61B 3/028; A61B 3/032**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Essilor International**
**94220 Charenton-Le-Pont (FR)**

(72) Inventors:
• **HERNANDEZ-CASTANEDA, Martha**
**75012 PARIS (FR)**

• **PETIGNAUD, Cécile**
**94800 VILLEJUIF (FR)**
• **MARIN, Gildas**
**75012 PARIS (FR)**
• **BAILLON, Loïc**
**75011 PARIS (FR)**
• **JOUARD, Ludovic**
**75011 PARIS (FR)**
• **GOULET, Alain**
**94490 Ormesson sur Marne (FR)**

(74) Representative: **Jacobacci Coralis Harle**
**32, rue de l'Arcade**
**75008 Paris (FR)**

(54) **ACCESSORY DEVICE FOR TESTING A SUBJECT'S EYE IN CONDITIONS OF NEAR OR INTERMEDIATE VISION**

(57)    The invention relates to an accessory device (2) for testing a subject's eye (E1, E2) in conditions of near or intermediate vision, designed to be used with an optometry device (1) for measuring a subjective value of refraction of the eye (E1, E2), said optometry device (1) comprising:

- a refraction test unit (10) having an optical component (11A, 12A) for providing different refraction powers to the eye (E1, E2) of the subject during a subjective refraction test, and

- a display unit (20) adapted to produce a visual target (T1, T2) for the subject's eye (E1, E2), an image of said visual target (T1, T2) being visible through said refraction test unit (10) along a reference observation direction (OBS1),

said accessory device (2) comprising an optical part (40) configured to be mounted on the refraction test unit (10) of the optometry device (1), comprising an optical system (41, 42) configured to deviate the image of the visual target (T1, T2) from said reference observation direction (OBS1) towards an inclined observation direction (OBS2, OBS3) along which the image of the visual target (T1, T2) is visible through the refraction test unit (10) and the optical part (40) of the accessory device (2), said inclined observation direction (OBS2, OBS3) defining a non-zero angle (A1, A2) with said reference observation direction (OBS1) when the optical part (40) is mounted on said refraction test unit (10) of the optometry device (1) and corresponding to a downward gaze direction of the subject and/or to a convergent gaze direction of the subject.

**Fig.2**

EP 4 371 468 A1

**Description**

TECHNICAL FIELD OF THE INVENTION

The invention relates to an accessory device for testing a subject's eye. The invention also relates to an optometry system comprising such an accessory device used in association with a conventional optometry device and an associated method.

BACKGROUND INFORMATION AND PRIOR ART

Known devices and methods for testing an eye of a subject are usually dedicated to testing the eye in far vision conditions. In far vision conditions, the visual target shown to the subject to test his binocular far vision is usually placed at over 5 meters from the subject's eyes, preferably 3 to 5 meters.

However, the refraction features of the eyes in near and/or intermediary vision conditions may be different from the refraction features in far vision. This may be due, among other things, to the fact that the accommodation and convergence of the eyes are different in near and/or intermediary vision conditions. It is known that the required visual correction is not the same in near vision with horizontal gaze and with downward gaze.

It is also known that accommodation is not the same in binocular vision and in monocular vision, and that the accommodation changes as a function of gaze orientation and of the used vision (monocular or binocular).

Moreover, accurate values of the refractive features of the eye or visual correction power needed are usually obtained through subjective measurement methods using optometry devices adapted to perform such subjective determination of the refractive features of the eye.

Known methods for testing the eyes of a subject in near and/or intermediary vision are implemented with the subject wearing trial frames equipped with trial lenses. Such a method does not permit to accurately control the conditions of refraction (gaze inclination, posture, object, and eye-lens distance...) and does not allow to maintain the trial lenses centered in front of the eyes of the subject. Unwanted aberrations may be induced (oblique astigmatism due to the non-perpendicular incident angle of the gaze on the lenses), creating deviations detrimental for the determination of the visual correction needed by the subject.

Alternative known methods for testing the eyes of a subject in near and/or intermediary vision use an optometry device, also called phoropter, that will help guaranteeing the centering of the optics onto the visual axis, the eye-lens distance, the control of the posture and the object (visual target) distance, avoiding the unwanted aberrations.

Such a phoropter comprises a refraction test unit and a support element designed to receive the head of the individual and to hold it in a predetermined position relative to the refraction test unit.

This refraction test unit houses one or more trial lenses providing different visual corrections that can be successively placed in front of the subject's eye until a suitable visual correction is found.

In practice, a plurality of trial lenses may be situated on two discs that are mounted to be free to rotate. Consequently, the trial lenses of this disc can successively be positioned in front of the corresponding subject's eyes. Alternatively, a single lens with variable power may be placed in front of the subject's eye, optionally associated with other lenses.

Phoropters usually only allow measurement with a straight-ahead gaze direction of the subject leading to non-natural posture of near and/or intermediary vision.

Some phoropter may be used with downward or convergent gaze directions of the subject. However, none of the existing phoropters allows determining a subjective value of the eye refraction in downward gaze without any complex and fastidious positioning steps. Indeed, such a determination requires rotating the refraction unit of the phoropter to place it in front of the eyes of the subject looking downward. The refraction unit of known phoropters rotates around an axis that is far above the eyes, making the inclination inducing a large change in the height of the phoropter, thus needing a complete readjustment of the position of the subject. Moreover, the ergonomics of a phoropter does not allow downward gaze while maintaining an appropriate alignment of eyes and phoropter's optics in comfortable conditions: the shape of the phoropter often pinch the nose or lean on the cheekbones of the subject. Besides, the conditions of the near and/or intermediary vision test are not comfortable for the patient and not easy to control for the eye care practitioner.

SUMMARY OF THE INVENTION

Therefore, the object of the invention is to provide an accessory device which can be used with an optometry device for testing a subject's eye in conditions of near or intermediate vision, in visual conditions that are comfortable and close to natural near or intermediary vision, without tedious adjustments.

This is achieved according to the invention by providing an accessory device for testing a subject's eye in conditions of near or intermediate vision, designed to be used with an optometry device for measuring a subjective value of refraction of the eye, said optometry device comprising:

- a refraction test unit having an optical component for providing different refraction powers to the eye of the subject during a subjective refraction test, and
- a display unit adapted to produce a visual target for the subject's eye, an image of said visual target being visible through said refraction test unit along a reference observation direction,
  said accessory device comprising an optical part configured to be mounted on the refraction test unit of the optometry device, comprising an optical system configured to deviate the image of the visual target from said reference observation direction towards an inclined observation direction along which the image of the visual target is visible through the refraction test unit and the optical part of the accessory device, said inclined observation direction defining a non-zero angle with said reference observation direction when the optical part is mounted on said refraction test unit of the optometry device and corresponding to a downward gaze direction of the subject and/or to a convergent gaze direction of the subject.

This accessory device is designed to be used with a conventional optometry device.

Thanks to the accessory of the invention, it is then possible to use a conventional optometry device to easily perform a subjective visual test of an eye of a subject in near and/or intermediary vision conditions, even if the conventional optometry device by itself only allows far vision measurements.

By mounting the accessory device of the invention on the optometry device it is indeed possible to modify an optical path along which the light is guided from the visual target to the subject's eyes. Within the optometry device, in the absence of the accessory device, the light is guided along a reference optical path corresponding to a horizontal straight ahead gaze direction of the subject in far vision conditions. The accessory device changes the optical path along which the light is guided into a final optical path. On the final optical path, light is deviated from the reference optical path towards a direction differing from the reference optical path by a non-zero angle and corresponding to a downward gaze direction and/or to a convergent gaze direction of the subject in near or intermediary vision conditions.

The downward gaze direction forms a non-zero angle with the horizontal straight ahead reference gaze direction in a vertical plane passing through said reference gaze direction. The convergent gaze direction forms a non-zero angle with the horizontal straight ahead reference gaze direction in a horizontal plane passing through said reference gaze direction.

In other words, an incident ray of light entering the accessory device along a horizontal straight incident direction is deflected by the optical system and exits the accessory device along an inclined exit direction forming a non-zero angle with said horizontal straight incident direction at least in one of a horizontal and a vertical plane, each passing through said reference gaze direction.

Thanks to the invention, it is possible to test a subject's eyes for subjective refraction with downward gaze and/or convergent gaze using the optometry device initially configured to determine a vision correction power with straight ahead gaze direction of the subject.

According to further non limiting features of the device of the invention:

- said optical system comprises a reflective or semi-reflective surface and/or an optical lens;
- said optical system comprises two optical elements, a first one of said two optical elements being configured to be placed at an active position in front of an exit aperture of said optometry device to deflect said image of the visual target and convey it to a second of said two optical elements, said second optical element being configured to deflect the image of the visual target along said inclined observation direction ;
- said first optical element is movable between said active position where it is placed in front of the optical component, on said reference observation direction of said optometry device and a second position where it is placed outside of said reference observation direction;
- said accessory device comprises a determination tool configured to determine a vision correction power for the eye of the subject, taking into account a final value of a parameter representative of the relative position of the eye and the optical component of said optometry device in the presence of said accessory device;
- said determination tool is configured to determine said vision correction power taking into account refraction powers provided by the refraction test unit of the optometry device during said subjective refraction test;
- said determination tool is configured to determine said vision correction power depending on a value of a parameter representative of the relative position of an image of the visual target seen through the accessory device and the eye of the subject;
- said determination tool is configured to determine said vision correction power depending on a reference value of a parameter representative of the relative position of the eye and the optical component of said optometry device in the absence of said accessory device; for example, a reference value of an optical vertex distance between the eye and the optical component of said optometry device;

- said determination tool comprises a software part programmed to calculate said vision correction power;
- said optometry device comprises a control device programmed to determine a reference vision correction power for the eye of the subject taking into account said refraction powers provided by the refraction test unit to the eye of the subject and a reference value of a parameter representative of a relative position of the eye and the optical component in the absence of said accessory device, and said software part is configured to be implemented by said control device of the optometry device;
- the accessory device comprises an adjustment tool configured to adjust a position and/or orientation of each visual target displayed by said display unit based on the inclination of said inclined observation direction and/or to adjust the refraction powers of the optical component of the optometry device based on a value of a parameter representative of the relative position of the image of the visual target and the eye of the subject;
- said determination tool comprises a database comprising data relative to said vision correction power for the eye of the subject linked to values of said parameter representative of the relative position of the eye of the subject and the optical component of the optometry device in the presence of said accessory device;
- said optical part comprises a housing where said optical system is housed, said housing being configured to be attached to the refraction test unit of the optometry device;
- said refraction test unit of the optometry device having two optical components for providing different refraction powers to both eyes of the subject, said optical part of the accessory device includes two optical systems each configured to be placed in front of one of said two optical components of said optometry device, allowing testing the eyes of the subject with binocular vision, each optical system comprising said first and second optical elements, the two first optical elements extend in a same first plane and the two second optical elements extend in a same second plane, the relative position and/or orientation of said two first optical elements and the relative position and/or orientation of said two second optical elements being fixed; and,
- said refraction test unit of the optometry device having two optical components for providing different refraction powers to both eyes of the subject, said optical part of the accessory device includes two optical systems, each optical system being configured to be placed in front of one of said two optical components of said optometry device, allowing testing the eyes of the subject with binocular vision, each optical system comprising said first and second optical elements, the position and/or orientation of each first optical element of said optical systems are adjustable independently from one another and the position and/or orientation of each second optical element of said optical systems are adjustable independently from one another;
- said refraction test unit of the optometry device having two optical components for providing different refraction powers to both eyes of the subject, said optical part of the accessory device includes two optical systems configured to be placed in front of said two optical components of said optometry device, allowing testing the eyes of the subject with binocular vision, each optical system comprising said first and second optical elements, the position and/or orientation of each first optical element of said sets being adjustable independently from one another and the relative position and/or orientation of the second optical elements of said sets being fixed;
- said software part is configured to determine the vision correction power based on the reference vision correction power, the reference value of the distance between the eye of the subject and the optical component of the optometry device and the final value of the distance between the eye of the subject, and the optical component;
- said software part is configured to determine the vision correction power taking into account the distance between the image of the visual target seen through the optometry device and accessory device and the eye of the subject;
- said vision correction power corresponds to an equivalent power at a predetermined standard distance between the eye and the optical component of the optometry device;
- said vision correction power is calculated with a predetermined formula or with a ray tracing algorithm;
- said software part is configured to adjust a position and/or orientation of each visual target displayed by said display unit based on the inclination of said inclined observation direction.

The invention also relates to an optometry system comprising an accessory device as described above, and an optometry device for measuring a subjective value of refraction of the eye, said optometry device comprising:

- a refraction test unit having an optical component for providing different refraction powers to the eye of the subject, and
- a display unit adapted to produce a visual target for the subject's eye, an image of said visual target being visible through said refraction test unit of the optometry device, along a reference observation direction, wherein said optical part of the accessory device is mounted on said refraction test unit.

Said optical system is configured to deviate the image of the visual target from said reference observation direction towards an inclined observation direction defining a non-zero angle with said reference observation direction.

Advantageously, said optical system is configured to be positioned in a predetermined fixed position relative to the eye of the subject where a center of rotation of the eye is located on the reference observation direction and on the inclined observation direction.

The invention also proposes a method for testing a subject's eye in conditions of far vision and near or intermediate vision with an optometry system as described above, said method comprising:

- performing a visual test in far vision conditions while the subject observes the image of one or more visual targets displayed by the display unit of the optometry device along said reference observation direction, non-deflected by the accessory device,
- using said accessory device to deflect said image of the visual target towards said inclined observation direction,
- performing a visual test in near or intermediate vision conditions while the subject observes the image of the visual target displayed by the display unit of the optometry device and deflected by the accessory device, along said inclined observation direction, without moving the refraction test unit between performing said visual test in far vision and performing said visual test in near or intermediate vision.

Advantageously, according to the method of the invention, it is also possible to perform the visual test in near or intermediate vision conditions without moving the head of the subject.

DETAILED DESCRIPTION OF EXAMPLE(S)

The following description with reference to the accompanying drawings will make it clear what the invention consists of and how it can be achieved. The invention is not limited to the embodiments illustrated in the drawings. Accordingly, where features mentioned in the claims are followed by reference signs, such signs are included solely for the purpose of enhancing the intelligibility of the claims and are in no way limiting on the scope of the claims.
In the accompanying drawings:

- figure 1 is a schematic view of an optical arrangement of an optometry device according to the state of art being used to measure the refraction of a subject's eye in far vision conditions,
- figure 2 is a schematic view of an optical arrangement of an optometry system according to the invention, comprising an accessory device coupled to the optometry device of figure 1, configured to measure the refraction of a subject's eye in near or intermediary vision conditions with downward gaze,
- figure 3 is a schematic view of the optometry system of figure 2 configured to measure the refraction of the subject's eye in far vision conditions, with straight ahead horizontal gaze direction,
- figure 4 is a schematic view of the optometry system of figure 2, seen from above, configured to measure the refraction of a subject's eye in near or intermediary vision conditions with convergent gaze,
- figure 5 is a schematic view of the optometry system of figure 2 showing a first embodiment of the accessory device of the invention mounted on the optometry device,
- figure 6 is an enlarged front view of the accessory device of figure 5 mounted on the optometry device,
- figure 7 is a schematic profile view of the subject looking through the accessory device of figure 5, with the accessory device configured to measure the refraction of the subject's eye in far vision conditions,
- figure 8 is a schematic profile view of the subject looking through the accessory device of figure 5, with the accessory device configured to measure the refraction of a subject's eye in near or intermediary vision conditions with downward gaze,
- figure 9 is a schematic front view of a second embodiment of the accessory device of the invention,
- figure 10 is a schematic view of the optometry system of the invention with a first variant of the accessory device, wherein said accessory device comprises two optical systems adapted to be placed in front of one of the eyes of the subject each with fixed global position and orientation relative to the optometry device, and each comprising a first and a second optical element, the first optical elements of both optical systems being made of a single piece and the second optical elements of both optical systems being made of a single piece,
- figure 11 is a schematic view of the optometry system of the invention with a second variant of the accessory device, wherein said accessory device comprises two optical systems, each one of them placed in front of one of the eyes of the subject, the optical elements of the two optical systems being separated from each other but remaining in a fixed position and orientation relative to the optometry device, not necessarily aligned with the optical axis of the optical component of the optometry device,
- figure 12 is a schematic view of the optometry system of the invention with a third variant of the accessory device, wherein said accessory device comprises two optical systems, each one of them placed in front of one of the eyes of the subject, the two optical systems being separated from each other and independently linked with a part of said optometry device, wherein the optical elements of each optical system are aligned with the optical axis of the optical component of the optometry device,
- figure 13 is an example of visual targets as displayed by the optometry device,

- figure 14 represents the image of the visual targets of figure 13 seen by each subject's eye when the accessory device is paired with the optometry device in the configuration represented in figure 12,
- figure 15 is the superposition of both images of the visual targets of figure 14,
- figure 16 is an example of visual targets corrected by the software part of the accessory device when the accessory device is coupled to the optometry device in the configuration represented in figure 12,
- figure 17 is the superposition of both images of the visual targets of figure 16 seen by the eyes of the subject through the accessory device,
and
- figure 18 is an optical scheme of the optical arrangement of the optometry system and the subject's eye used for calculating the vision correction power.

In the following description, identical or corresponding elements of embodiments and variants of the invention will be designated by the same references.

The direction of propagation of light is indicated by arrowheads.

The invention relates to an accessory device 2 for testing a subject's eye in near or intermediate vision conditions when used with an optometry device 1.

It also relates to an optometry system 3 comprising the optometry device 1 and the accessory device 2 mounted on it.

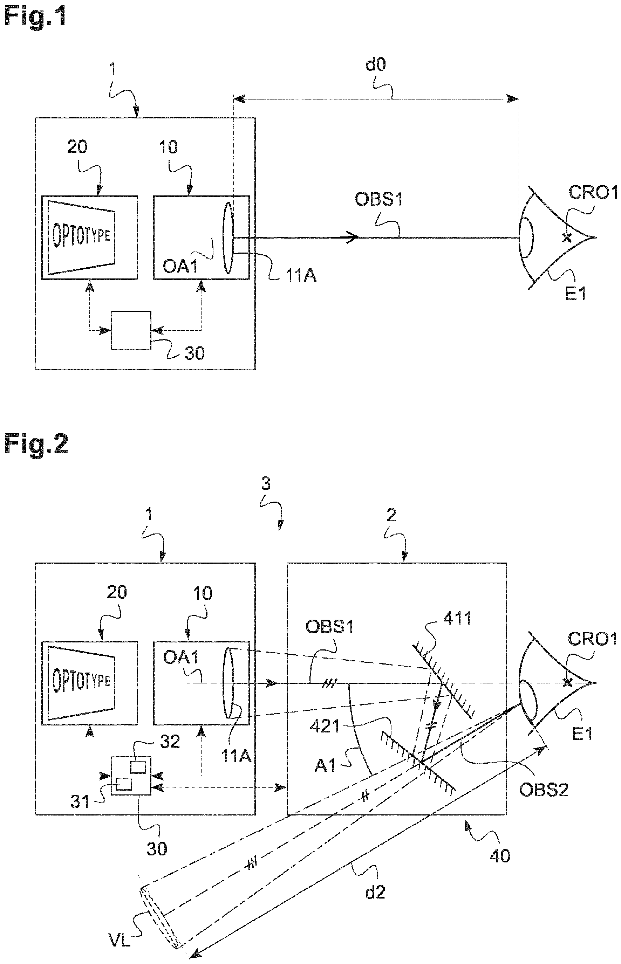

The optometry device 1 is configured for measuring subjective values of the refraction of the eye by determining a reference vision correction power needed by the subject's eye to achieve a target visual performance, as explained in more details in the following. Figure 1 shows a schematic view of an optometry device according to the state of the art, without the accessory device 2. Figures 2 to 4 and 6 to 12 show schematic views of an optometry system 3 of the invention, comprising an optometry device 1 similar to the one of figure 1 and the accessory device 2 of the invention.

Typically, said subjective test comprises several steps during each of which the subject is required to compare two different optical situations taking into account a test value of an optical feature, such as spherical and/or cylindrical power and/or axis, of an optical component of the optometry device. A subjective test therefore corresponds to a sequence of steps. Depending on the feedback of the subject on this comparison (the answer of the subject), the eye-care professional increments the test value and, in the next step of the subjective test, presents the subject with two new different optical situations based on the incremented test value. This process is repeated until a specific answer or combination of answers is given by the subject. An example of such an optometry device 1 is represented on figures 1 to 12.

The optometry device 1 is commonly called "a phoropter". It is a conventional optometry device and can be of any type known from the man skilled in the art. It may be manual or automatic. It may comprise a virtual reality headset comprising a light field display.

Such an optometry device 1 and its use in a subjective refraction test are known and only their main features will be described in the following.

As represented schematically on figures 1 to 3, the optometry device 1 comprises a refraction test unit 10 and a display unit 20. The optometry device 1 is commonly used to test a subject's eyes preferably in far vision conditions.

Known subjective refraction tests may be implemented, with binocular or monocular vision conditions. Subjective tests for determining a vision correction power may be preferably performed with binocular vision conditions. A full refraction subjective test or a subjective test for determining the addition may be performed in near vision conditions with said reference observation direction OBS1.

The refraction test unit 10 of the optometry device 1 comprises at least an optical refraction element 11, 12 for providing different refraction powers to the eye of the subject.

The optometry device 1 is preferentially used to make binocular measurements determining at least one refraction feature of a first eye E1 and/or of a second eye E2 of a subject, in a binocular manner.

However, it could be used for monocular measurements. A phoropter with one optical refraction element and one image displayed can be considered for monocular measurements.

The binocular determination of the refraction feature of an eye is based on a binocular measurement performed while the subject has both eyes E1, E2 opened and un-obstructed.

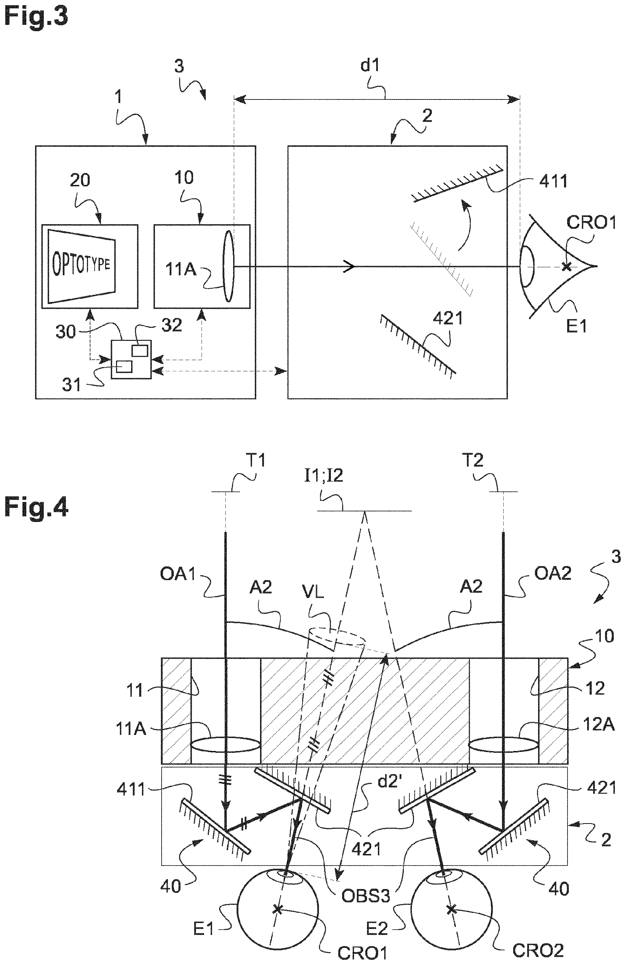

Said refraction test unit 10 of the optometry device 1 therefore has two optical refraction elements 11, 12 for providing different refraction powers to both eyes E1, E2 of the subject, as shown on figure 4.

The refraction test unit 10 comprises a first optical refraction element 11 adapted to provide different vision correction powers along a first optical axis OA1 and a second optical refraction element 12 adapted to provide different vision correction powers along a second optical axis OA2 (figures 4 and 10-12).

Said first optical refraction element 11 is configured for providing the first eye E1 of the subject with a first correction power and said second optical refraction element 12 is configured for providing the second eye E2 of the subject with a second correction power.

In the optometry device 1 shown on the appended figures, and described in the following, each of said first and second optical refraction elements 11, 12 comprises at least an optical component 11A, 12A such as a lens or a mirror or a prism, or a set of such optical components that has adjustable refractive power features or permits to adjust refractive power by rotation or translation of the optical components or by an electrical command.

In practice, the optical component 11A, 12A comprises for example a lens with variable power. It comprises here a deformable liquid lens having an adjustable shape. The optical axis OA1, OA2 mentioned before thus correspond to the optical axis of the corresponding lens.

Each optical component 11A, 12A may comprise a lens with variable spherical power and a lens with a variable cylindrical power and variable cylindrical axis. It may also comprise a prism or any other component adapted to provide prismatic power to the eyes of the subject. The resulting optical component is schematically represented on the figures by a lens 11A, 12A (figures 1-4, 10-12).

Alternatively, or in addition, the optical component may comprise an ensemble of non-deformable lenses having different optical powers, and a mechanical system that enables to select some of these lenses to group them to form the set of lenses through which the subject can look. In this last case, to adjust the refractive power of the set of lenses, one or several lenses of the set of lenses are replaced by other lenses stored in the refraction test unit. The optical axis mentioned before thus corresponds to the optical axis of the lens placed in front of the eye of the subject.

Each of the optical refraction element 11, 12 is intended to be placed in front of one of the eyes E1, E2 of the subject, close to this eye.

A relative position between the subject's eye and the corresponding optical component 11A, 12A of the optical refraction element 11, 12 is quantified by a value of the parameter representative of this relative position of the subject's eye E1, E2 and the optical component 11A, 12A of the refraction test unit 10.

Values of the parameter representative of this relative position of the subject's eye E1, E2 and the optical component 11A, 12A of the refraction test unit 10 may either be measured, calculated, estimated, or predetermined.

This parameter may be a distance measured between the optical component 11A, 12A of the optical refraction element 11, 12 turned towards the eye and the eye E1, E2 placed in front of it, for example between the apex of the curvature of the outside face of the optical components 11A, 12A and the apex of the curvature of the cornea of the eye.

More precisely, said parameter may be an optical vertex distance defined as the optical distance between the rear surface of the lens of the optical component 11A, 12A and the apex of the cornea of the eye.

The distance measured may be a physical distance or an optical distance. The physical distance between two points corresponds to the distance measured along a straight line between the two points. The optical distance is measured along the optical path of light between the two points. The optical distance may or may not take into account the index of the material where the path of light is located. The optical distance between two points may then correspond to the physical distance measured between two points while following the optical path of light from one point to the other.

For example, it is equal to the sum of the physical distances measured between consecutive intersection points between the path of light with the surfaces of the optical components. When the index of the material is taken into account, the optical distance is equal to the physical distance multiplied by the index. In practice, when light goes through the air and/or different materials, the optical distance is equal to the sum of the physical distance traveled within the air multiplied by the refractive index of the air and/or the sum of the physical distances traveled within said different materials, each physical distance being multiplied by the refractive index of the corresponding material.

The parameter may also for example be defined as the optical distance between the eye rotation center and the optical component, without taking into account the index of the materials through which the light travels.

This parameter may also be an angle.

The value of said parameter may for example be manually measured, determined based on an image of the head of the subject and the optometry device or predetermined as equal to an average value. The accessory device 2 may comprise measuring devices, as described in more detail hereafter.

The optometry device 1 allows determining said reference vision correction power for the eye E1, E2 of the subject taking into account said refraction powers provided by the refraction test unit 10 to the eye of the subject and a reference value of said parameter representative of the relative position of the eye and the optical component in the absence of said accessory device 2, as detailed hereafter. The reference vision correction power corresponds to a reference value of the vision correction power needed by the subject's eye to achieve a target visual performance determined with the reference value of said parameter representative of the relative position of the subject's eye E1, E2 and the optical component 11A, 12A of the refraction test unit 10.

The relative position of the eye of the subject and the optical component 11A, 12a of the optometry device 1 is for example controlled by using one or more position adjustment elements adapted to receive the head of the subject.

Said one or more position adjustment elements may belong to the refraction test unit 10. They may be designed to hold the head of the subject in a given position relative to the refraction test unit 10.

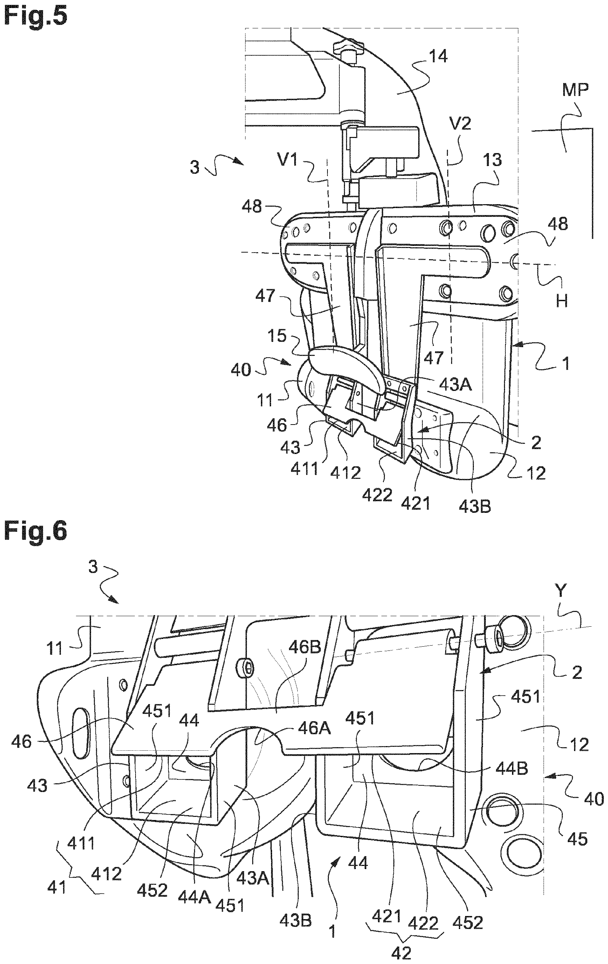

The refraction test unit 10 comprises for example a position adjustment element 15 adapted to receive the forehead of the subject (see figure 5). Alternatively, or in addition, the refraction test unit could comprise an element adapted to receive the chin of the subject.

The position adjustment element may then be used to obtain a predetermined value of the parameter representative of the relative position of the eye and the optical component 11A, 12A.

The position adjustment elements are for example configured to ensure that the optical vertex distance between the eye of the subject and the optical component 11A, 12A of the optometry device 1 in the absence of the accessory device 2 is predetermined and equal to a target value of the optical vertex distance equal to 12 mm.

In the following, we will describe the optometry device 2 in the case where each optical component 11A, 12A comprises a lens with variable spherical power and a lens with a variable cylindrical power and variable cylindrical axis and, optionally, a prism, represented on the figures by the lens with the reference 11A, 12A.

The optical component 11A, 12A has an overall spherical power S corresponding to the spherical optical power, expressed in diopters. The optical component 11A, 12A has a cylindrical power C expressed in diopters and an orientation represented by an angle A. Each of the first and second refraction power, provided by the corresponding optical refraction element 11, 12, may be characterized by the values of these three refractive power parameters S, C and A.

Said optical refraction elements 11, 12 are mounted on a common support 13 (figure 5) that extends between the optical refraction elements 11, 12, above them, along a longitudinal axis H (figure 5) that is horizontal.

This support 13 is linked to a global supporting structure 14 (partially represented figure 5) that lies on a table or on the ground.

Each of the optical refraction elements 11, 12 is mounted on the support 13 to be mobile in rotation about a rotation axis V1, V2 perpendicular to said longitudinal axis H (figure 5). A mean plane MP of the first and second optical refraction elements 11, 12 goes through these axes V1, V2 (figure 5). This mobility of the optical refraction elements allows adjusting the orientation of the optical axes OA1, OA2 of the optical components 11A, 12A. In particular, the optical components 11A, 12A may then be oriented to take into account the convergence of the eyes of the subject

The optometry device 1 also comprises a display unit 20 for providing one or more visual targets T1, T2. Said display unit 20 is adapted to produce a visual target for the subject's eye, an image I1, I2, I1', I2' of said displayed visual target T1, T2 being visible through said refraction test unit 10 along the reference observation direction OBS1. This reference observation direction OBS1 extends along the optical axis OA1, OA2 of the optical component 11A, 12A of the refraction test element 11, 12 (figure 1). The reference observation direction OBS1 corresponds to a horizontal straight ahead gaze direction. The image of the visual target is visible through an exit aperture of the optical refraction element 11, 12 of the optometry device 1.

The image of the visual target may be the visual target itself when it is looked at directly by the subject. It may also be a real or virtual image of the visual target through the optical setup of the optometry device 1, and, optionally, of the accessory device 2 when it is mounted on said optometry device 1.

It preferentially provides a first and a second visual targets T1, T2 for binocular tests. The image I1, I1' of the first visual target is conveyed to the first optical refraction element 11 along a first optical pathway and the image I2, I2' of the second visual target is conveyed to the second optical refraction element 12 along a second optical pathway.

The image display unit 20 is thus configured for providing an image of the first visual target to the first eye E1 of the subject and, at the same time, for providing an image of the second visual target to the second eye E2 of the subject. The first and second visual targets may be the same or may be different from each other.

The image of the first visual target is seen by the first eye E1 of the subject through the first optical refraction element 11, while the image of the second visual target is seen by the second eye E2 of the subject through the second optical refraction element 12.

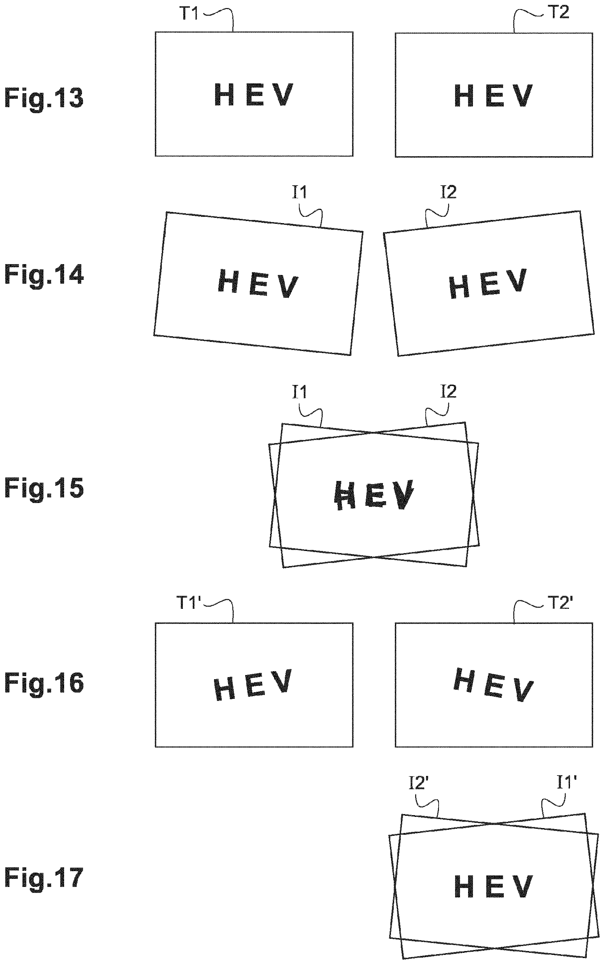

The images I1', I2' of the visual targets T1, T2 provided to both eyes E1, E2 are preferably configured such that the fusion of the two visual targets by the brain of the subject may occur (figure 17). Preferably, the two visual targets are stereoscopic images providing a representation at least partially in three dimensions for the subject.

In order to achieve this end, each visual target is configured to be accurately optically aligned with the corresponding eye of the subject.

Preferably, said first and second optical pathways allow testing the eyes of the subject in far vision. The optical distance corresponding to said optical pathways is preferably over 1.5 meters, even more preferably between 3 and 5 meters.

The display unit 20 may comprise a printed visual target. In another embodiment, it can comprise one or more electronic devices, each comprising a screen and/or an element adapted to display a visual target. The screen is for instance one of the following: a LED or OLED 20 screen, a serigraphy with backlight screen, a display light projection screen with micro video projector, an LCD screen or a TFT screen. The display unit 20 then comprises an active screen which produces a light beam.

In another embodiment, the display unit comprises a projector and a projection element adapted to project one or more visual targets. By projected, it is meant that each visual target is formed by the projection element, such as a lens.

Each visual target may be projected on a passive screen or directly on the retina of the eye of the subject.

Each visual target T1, T2 comprises for example one or more optotypes (figure 13). Each visual target may comprise any kind of visual target adapted to test the vision of the subject known of the man skilled in the art may be used.

Preferably, the optometry device 1 also comprises a control device 30. The control device 30 may comprise a processor and an interface such as a screen. It is programmed to determine the reference vision correction power for the eye of the subject based on the subjective test performed with the optometry device 1.

The control device 30 is programmed to determine the reference vision correction power for the eye E1, E2 of the subject taking into account said refraction powers provided by the refraction test unit 1 to the eye E1, E2 of the subject. It may also take into account the reference value of the parameter representative of a relative position of the eye E1, E2 and the optical component 11A, 12A in the absence of said accessory device 2.

Such control device 30 is well-known and will not be described in more details here.

As mentioned above, the optometry device 1 is configured to place the optical component 11A, 12A at a reference position relative to the subject's eye. This reference position of the optical component relative to the subject's eye corresponds to the reference value of said parameter representative of the relative position of the eye and the optical component of said optometry device without the presence of said accessory device.

The subjective test is performed at said reference value of the parameter representative of the relative position of the eye and the optical component of said optometry device to determine said reference vision correction power.

The reference value d0 of the optical vertex distance without the accessory device corresponds to the optical distance between the apex of the cornea of the eye of the subject and the apex of the rear surface of the optical element 11A, 12A measured along the reference observation direction OBS1 (figure 1). It is here equal to the physical horizontal distance measured with the eye in the primary position, i.e., with a straight-ahead horizontal gaze direction.

The reference value d0 of the optical vertex distance used in the optometry device 1 without the accessory device 1 is usually equal to 12 mm.

The reference value d0 of the optical vertex distance may alternatively be comprised for example between 11 and 15 mm.

In practice, the reference vision correction power is usually determined as a function of the test values of the refraction feature of the optical component used during said subjective test, corresponding to an optimum for visual correction of the visual defect of the eye of the subject.

By vision correction power, it is meant a dioptric power allowing correcting a refraction error of the eye of the subject, such as sphere power, cylinder power and axis, prismatic power, and axis.... Values of a plurality of vision correction powers comprising values of a plurality of different dioptric powers may also be determined.

The optometry device 1 without accessory device 2 is mostly used for measurement performed with a straight-ahead horizontal gaze direction of the subject. Using this conventional optometry device for measurements with a downward gaze and/or convergent gaze would require a long positioning process to ensure accurate alignment of the eyes of the subject and the optical components of the optical refraction elements. Moreover, the position of the refraction test unit 10 and/or of the head of the subject would be necessarily modified.

In order to easily test the subject's vision with a downward and/or convergent gaze direction, the accessory device 2 can be installed on the optometry device 1 to obtain the optometry system 3.

The gaze direction of one eye of the subject corresponds to the straight lines going through at least two of the following points: a center of rotation of the eye, a pupil center of the eye and a center of the visual target.

In figure 2, the accessory device 2 is represented coupled to an optometry device 1 as described above. A subject's eye is placed in front of the accessory device 2.

The accessory device 2 is used to deviate the image I1, I2 of each visual target T1, T2 displayed by the display unit 20 of the optometry device 1 from said reference observation direction OBS1 towards an inclined observation direction OBS2, OBS3 (figures 2 and 4). The inclined observation direction is oriented downwards (figure 2) and/or towards the other eye (convergent) (figure 4).

A distance of far vision is typically comprised between infinity and 1,5 meters with a straight horizontal gaze direction corresponding to a reference angle of 0°. A typical distance of far vision may be comprised between 3 and 5 meters. A distance of intermediate vision is typically comprised between 150 and 50 centimeters with approximatively a 15° downward gaze direction. A distance of near vision is typically comprised between 50 and 25 centimeters with approximatively a 30° downward gaze direction.

The inclined observation direction OBS2 is for example oriented at a downward angle A1 comprised between 15° to 45°, preferably equal to 30°, from the reference observation direction OBS1, measured in a vertical plane comprising this reference observation direction OBS1 (figure 2). The reference observation direction OBS1 usually corresponds to the horizontal straight ahead gaze direction. The inclined observation direction OBS2 then corresponds to a downwards near vision gaze.

The inclined observation direction OBS3 may also be oriented at a convergent angle A2 comprised between 2° to 15° from the reference observation direction OBS1, measured in a horizontal plane comprising said reference observation direction OBS1 (figure 4). The inclined observation direction OBS2 then corresponds to a convergent vision gaze.

According to the invention, the optometry device 1 may be used with the accessory device 2 for testing a subject's eye, with the following steps:

- performing a visual test in far vision conditions while the subject observes the image I1, I2 of one or more visual targets T1, T2 displayed by the display unit 20 of the optometry device 1 along said reference observation direction OBS1, non-deviated by the accessory device 2,
- using said accessory device 2 to deflect said image I1, I2 of the visual target T1, T2 towards said inclined observation direction OBS2, OBS3,
- performing a visual test in near or intermediate vision conditions while the subject observes the image I1, I2 of the visual target displayed by the display unit of the optometry device and deflected by the accessory device, along said inclined observation direction OBS2, OBS3, without moving the refraction test unit 10 between performing said visual test in far vision and performing said visual test in near or intermediate vision.

Said visual tests are subjective refraction tests as mentioned before. The visual perception of the visual target is assessed by asking the subject to characterize his perception of the visual target, by saying if it is seen or not seen, or by recognizing or not an optotype such as a letter. The visual perception may also be assessed, for example, by determining the delay needed by the subject to recognize an optotype. The assessment of the visual perception may be done by any method known from the person skilled in the art.

Further, the optical power of the lenses in the optometry device and/or the visual target are changed depending on the responses of the subject during the assessment step.

In said method, the visual test in far vision may be performed before or after the visual test in near or intermediate vision conditions. Preferably, it is performed before.

To this end, the accessory device 2 comprises an optical part 40. The optical 40 part is configured to be mounted on the refraction test unit 10 of the optometry device.

The optical part 40 comprises one or more optical systems 41, 42 configured to deviate the image of the visual target produced by said optometry device 1 from said reference observation direction OBS1 towards the inclined observation direction OBS2, OBS3.

The inclined observation direction OBS2, OBS3 defines a non-zero angle with said reference observation direction OBS1 when the optical part 40 is mounted on said refraction test unit 10 of the optometry device 1. In other words, said one or more optical systems 41, 42 are configured to deviate a light beam exiting the optometry device 1 from the reference observation direction OBS1 towards the inclined observation direction OBS2, OBS3.

As the optometry device 1 of the example is configured for binocular measurements, the refraction test unit 10 of the optometry device 1 has two optical refraction elements 11, 12 for providing different refraction powers to both eyes of the subject. Consequently, said optical part 40 of the accessory device 2 includes here two optical systems 41, 42, each optical systems 41, 42 being configured to be placed in front of one of said two optical refraction elements 11, 12 of said optometry device 1, allowing testing the eyes of the subject with binocular vision.

Each optical system 41, 42 comprises one or more optical element 411, 412, 421, 422.

In the example shown on figures 2 to 12, each optical system 41, 42 comprises two optical elements 411, 412, 421, 422.

Each optical element 411, 412, 421, 422 comprises for example a reflective or semi-reflective surface.

In the example shown on the appended figures 2 to 12, the two reflective surfaces belong to two mirrors. In other embodiments of the invention, the reflective surfaces may belong to prisms, semi-reflecting blades, spherical mirrors, aspherical mirrors, or any kind of appropriate optical elements known of the man skilled in the art.

A first one 411, 412 of said optical elements 411, 412, 421, 422 is configured to be placed at an active position in front of one of the exit apertures of said optometry device 1 to deflect said visual target and convey it to a second 421, 422 of said two optical elements 411, 412, 421, 422, said second optical element 421, 422 being configured to deflect the image of the visual target along said inclined observation direction OBS2, OBS3.

In the example described hereafter, each optical system 41, 42 comprises a first and a second optical elements 411, 412, 421, 422. In a variant, said optical element may comprise other optical elements positioned in between said first and second optical elements.

Figure 2 on one hand and figure 4 on the other hand show two different internal arrangements of the optical part 40 of the accessory device 2.

The internal arrangement of figure 2 shows a first relative position of the first and second optical elements 411, 412, 421, 422. In this first relative position, two planar mirrors corresponding to the two optical elements 411, 412, 421, 422 are placed partly one above the other along a vertical direction, at least partly facing each other.

As shown on figure 2, a first mirror 411, 421 of the accessory device 2 is adapted to be inclined relative to the reference observation direction OBS1. The first mirror 411, 421 may be inclined towards the second mirror 412, 422, with an inclination angle about a horizontal axis perpendicular to the reference observation direction OBS1, as compared with a vertical plane perpendicular to the reference observation direction OBS1. The second mirror 412, 422 is placed beneath the first mirror. With the first and second optical elements in the first relative position, the inclined observation direction OBS2 corresponds to a downward gaze direction of the eye of the subject.

The internal arrangement of figure 4 shows a second relative position of the first and second optical elements 411, 412, 421, 422. In this second relative position, the two mirrors corresponding to the two optical elements 411, 412, 421, 422 are placed side by side along a horizontal direction, partly facing each other.

As shown on figure 4, a first mirror 411, 421 of the accessory device 2 is adapted to be inclined relative to the reference observation direction OBS1. The first mirror 411, 421 may be inclined towards the second mirror 412,422, with an inclination angle about a vertical axis perpendicular to the reference observation direction OBS1, as compared with a vertical plane perpendicular to the reference observation direction OBS1. The second mirror 412, 422 is placed beside the first mirror, on the side closer to the other eye. With the first and second optical elements in the second relative position, the inclined observation direction corresponds to a convergent gaze direction of the eye of the subject.

The internal arrangements described above may be combined so that the inclined observation direction is both downwards and convergent.

It is to be noted that the convergent observation direction may be obtained at least partly by rotating the refraction test elements 11, 12 of the optometry device 1 about their rotation axes V1, V2. The optical systems of the accessory device 2 may then help to adjust the distance between the optical components of the optometry device to the interpupillary distance of the eyes of the subject. This is particularly useful for pupillary distances smaller than the average pupillary distance.

This case is shown on figure 4, where the interpupillary distance between the eyes of the subject is smaller than the distance between the optical axis OA1, OA2 of the optical components 11A, 12A of the optometry device 1. The accessory device 2 allows performing the subjective test on eyes having such an interpupillary distance.

It is also useful for accessory devices introducing a larger change in distance between the eye and the corresponding optical component.

The ratio between the convergence introduced by the accessory device 2 and the convergence introduced by the optometry device 1 may depend on the subject's pupillary distance: when the pupillary distance increases - in other words, for people with higher pupillary distance - the optometry device introduces an increasing part of the convergence.

When the pupillary distance of the subject decreases - in other words, for people with lower pupillary distance - the part of the convergence introduced by the optometry device decreases. Using both the optometry device 1 and the accessory device 2 to obtain the convergent observation direction also allows changing the distance between the virtual image of the visual target without modifying the configuration of the optical system of the accessory device.

The optometry system 3 of the invention can then be used for any pupillary distance value at any distance between eye an optical component of the optometry device and for any distance between the eye and the virtual image of the visual target.

Preferably, the size of the optical elements of the accessory device 2 is adjusted in order not to limit the field of view of the optometry device.

In order to ensure that the field of view of the eye of the subject is not restricted by the mirrors, basic geometrical optics of the mirrors is applied, as shown schematically on figure 2 and explained hereafter.

Figures 2, 4, and 8 show the optical path of a light ray emitted by the display unit 20 represented in full straight lines, starting from the visual targets T1, T2 (figure 4) or from the optical components 11A, 12A of the optometry device 1 to the eye E1 of the subject.

Figures 2, 4 and 8 show the optical path of light when the accessory device 2 is in a configuration where the ray of light is deviated from the reference observation direction OBS1 by the first optical component 411, 421 of the accessory device 2. The optical path of the light ray initially extends along the optical axis OA1 of the optical component 11A of the optometry device 1, which is aligned with the reference observation direction OBS1. The light ray is reflected by the first mirror 411 towards the second mirror 421 and reflected by the second mirror 421 towards the pupil center of the eye of the subject, along the final observation direction OBS2, OBS3 (figures 2, 4 and 8).

In a general manner, the center of rotation CRO1 of the eye E1 may advantageously be aligned with the reference observation direction OBS1 and preferably aligned with both the reference observation direction OBS1 and the inclined observation direction OBS2, OBS3 to ensure switching from looking at the image of the visual target along the reference observation direction OBS1 and looking at the image of the visual target along the inclined observation direction OBS2, OBS3 by simply removing the first mirror 411 from the reference observation direction OBS1 without changing the subject's eye position.

Figures 2, 4 and 8 show a schematic representation of an equivalent straight path of light represented in hashed lines. This equivalent straight path of light extends along the inclined observation direction OBS2, OBS3. A virtual position VL of the optical component 11A along this equivalent straight path of light is shown in hashed lines. This virtual position VL is obtained by unfolding the path of light between the optical component and the eye of the subject to trace the equivalent straight path of light (represented in hashed lines).

An optical vertex distance d2, d3 between the eye and the virtual position VL is equal to an optical vertex distance d3between the eye and the optical component 11A: it is for example the sum of the optical distance between the apex of the cornea of the eye and the second mirror 412 along the path of light, with the optical distance between the second 412 and the first mirror 411 along the path of light and the optical distance between the first mirror 411 and the apex of the face of the optical component 11A oriented towards the eye.

This unfolded representation of the path of light in the optometry device 1 and accessory device 2 allows to visualize the optical vertex distance in the downward or convergent gaze direction configurations of the optometry system 3 corresponding to the values of said parameter in downward or convergent gaze direction with the accessory device 2.

It also allows checking that the field of view is limited by the exit aperture of the optometry device 1, not by the mirrors. The dash dotted lines of figures 2 and 4 show that the full extent of the optical component is visible through the accessory device 2.

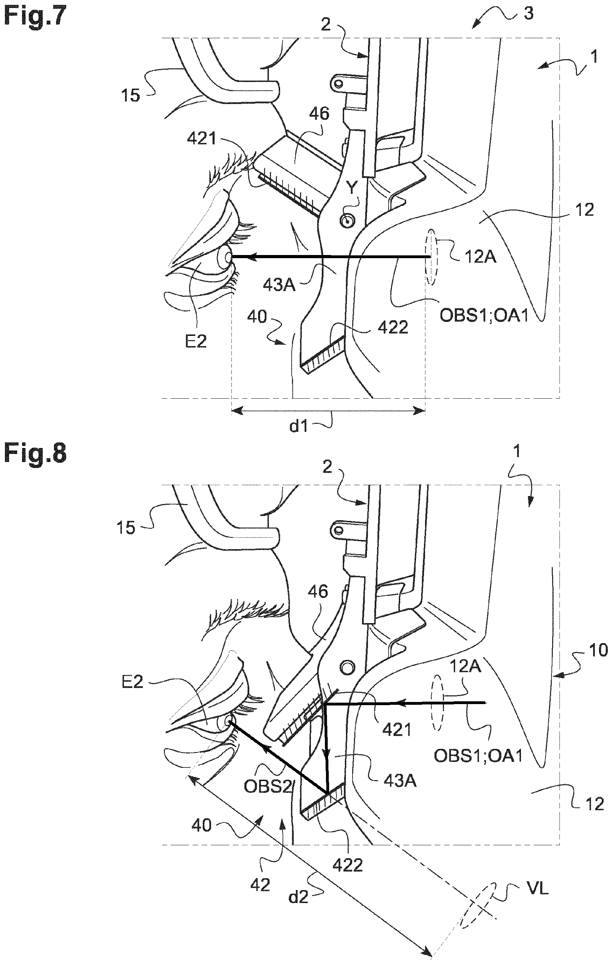

Advantageously, in an embodiment shown on figures 2, 3 and 5 to 8, said first optical element 411, 412 is movable between said active position where it is placed in front of the exit aperture of the optometry device 1, in other words in front of the optical component 11A, 12A, on said reference observation direction OBS1 of said optometry device 1 (figures 2 and 8) and a second retracted position where it is placed outside of said reference observation direction OBS1 (figures 3 and 7).

When the first optical element 411, 412 is in the active position, the light beam exiting the optometry device is intercepted by the first optical element 411, 412 and deflected towards the second optical element 421, 422. The image of the visual target is visible by the eye of the subject on the inclined observation direction OBS2, OBS3. The subjective test may be performed in conditions of near or intermediate vision, with a downward and/or convergent gaze direction.

When the first optical element 411, 412 is in the second retracted position, the light beam exiting the optometry device is not deflected by the accessory device and the image visual target is visible by the eye E1 of the subject on the reference observation direction OBS1 (figure 3). The subjective test may be performed in conditions of far vision with a horizontal straight ahead gaze direction.

This mobility of the first optical element allows testing the subject's eye in a downward gaze direction OBS2 and/or convergent gaze direction OBS3 and in a horizontal straight ahead gaze direction OBS1 without removing the accessory device 2 as a whole from the optometry device 1. Moreover, the subjective tests in far vision conditions and near or intermediate vision conditions may be performed without moving the optometry device 1.

The two optical elements 411, 412, 421, 422 of each optical system 41, 42 are preferably thus arranged so that both the reference observation direction OBS1 and the inclined observation direction OBS2, OBS3 go through the center of rotation CRO1, CRO2 of the eye E1, E2 of the subject when in use. The subjective tests in far vision conditions and near or intermediate vision conditions may then be performed without moving the refraction test unit of the optometry device nor the subject's head. This is made possible by taking into account a predetermined position of the head of the subject, controlled for example by position adjustment elements of the optometry device.

Alternatively, the accessory device could have fixed optical elements, such as fixed mirrors, with no mobility. In this case, the accessory device could be entirely removed from the optometry device 1 to use the optometry device with a straight-ahead horizontal gaze direction. In this case, easily removable attaching means for the accessory device may ensure that the tests with downwards and/or convergent gaze and horizontal straight-ahead gaze are performed without moving the optometry device 1.

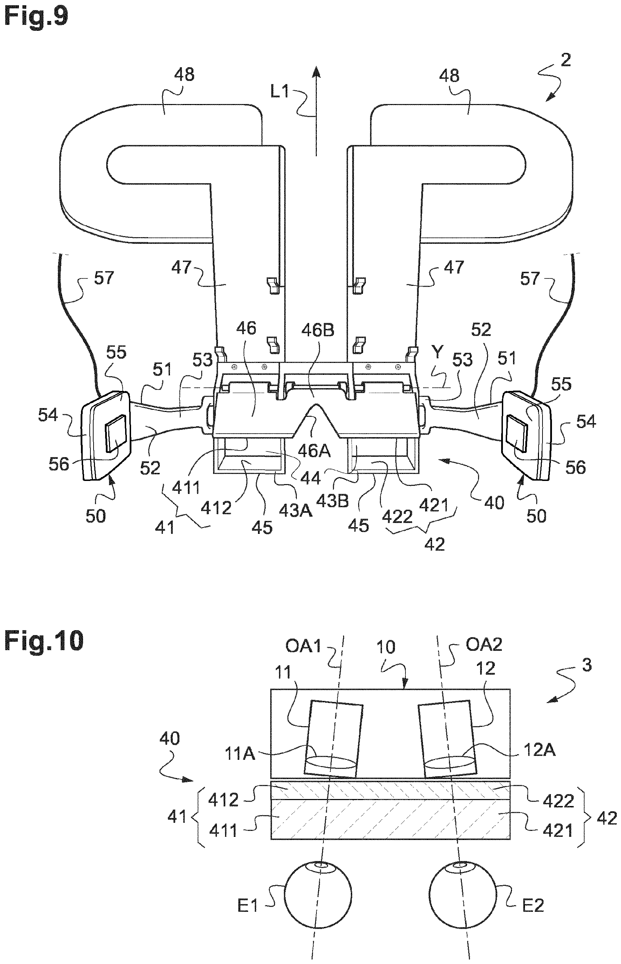

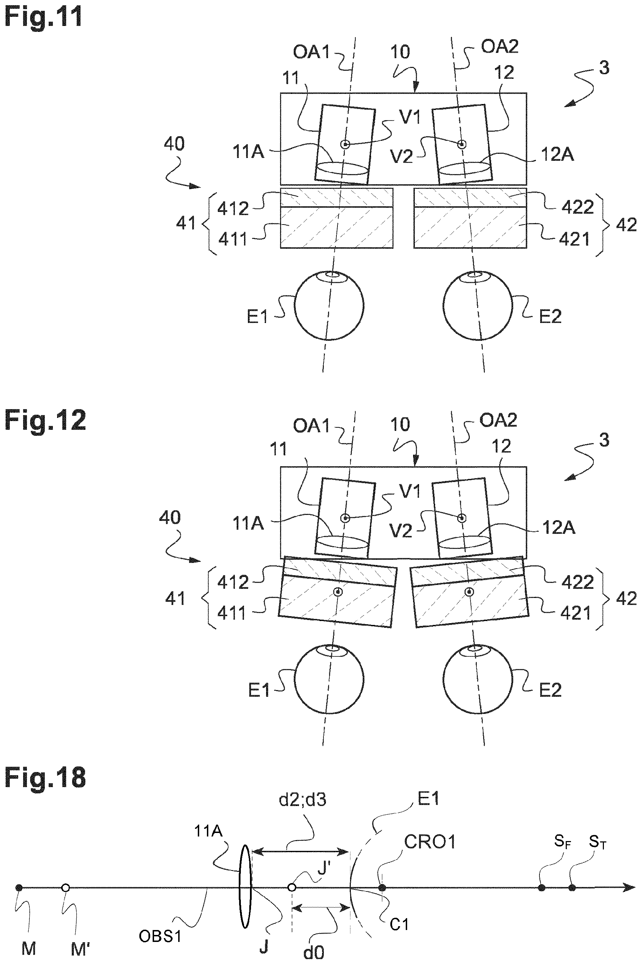

In a first and a second variant of the accessory device 2 of the invention, schematically represented on figures 10 and 11, the two first optical elements 411, 412 of the two optical systems 41, 42 of the accessory device 2 extend in a same first plane and the two second optical elements 421, 422 of the two optical systems 41, 42 of the accessory device 2 extend in a same second plane.

The relative positions and/or orientations of said two first optical elements 411, 412 and the relative positions and/or orientations of said two second optical elements 421, 422 are fixed. This implies that the two first optical elements 411, 412 are moved together when the first optical element are mobile as described above.

In particular, in the first variant represented on figure 10, the reflective or semi-reflective surfaces of the two first optical elements 411, 412 may belong to a single piece, for example to a first single mirror. The reflective or semi-reflective surfaces of the two second optical elements 421, 422 may also belong to a single piece, for example to a second single mirror. The first single mirror forming the two first optical elements 411, 412 may be mobile between the first and second retracted position as described above.

This ensures that the optical path of light is the same for both eyes and allows and accurate alignment of optical component of the optometry device, the optical system of the accessory device and the eye of the subject. The image of each visual target is seen by the corresponding eye and the fusion of the two images may occur without any alignment issue.

In practice, errors in the alignment of the mirrors should be less than 1°, preferentially less than 1' to ensure comfortable fusion and therefore accurate binocular vision of the visual target.

In the second variant represented on figure 11, the optical systems 41, 41 are formed by distinct first and second optical elements. However, the reflective or semi-reflective surfaces of the two first optical elements 411, 412 of the optical systems 41, 42 are aligned in the same plane. They may comprise for example two coplanar mirrors. The reflective or semi-reflective surfaces of the two second optical elements 421, 422 may are also aligned in another plane, for example belonging to two different coplanar mirrors.

The relative positions and orientations of the two optical systems 41, 42 as a whole are fixed. The positions and /or orientations of the two first optical elements on one hand and of the two second optical elements on the other hand are fixed or may only be changed conjointly.

In a third variant shown on figure 12, the position and/or orientation of each optical system 41, 42 is adjusted so that the optical systems 41, 42 remain aligned with the corresponding optical component 11A, 12A of the optometry device 1. The position and/or orientation of each optical system 41, 42 is adjustable independently from the position and/or orientation of the other optical system 41,42.

The relative position and orientation of the first and second optical elements of each optical system 41, 42 is fixed except for the optional mobility of the first optical element mentioned above.

In particular, each optical system 41, 42 may be rotated about a rotation axis X1, X2 (figure 12). This rotation axis X1, X2 is configured to be parallel to the rotation axis V1, V2 of the optical refraction elements 11, 12 of the optometry device 11, when the accessory device 2 is attached to the optometry device 1.

In this case, when the optical systems 41, 42 are rotated to be aligned with the converging optical axes OA1, OA2 of the optical components of the optometry device1, the accessory device comprises preferably a software part programmed to rotate symmetrically the visual targets displayed by the display unit 20 of the optometry device 1 for both eyes to compensate for the cyclorotation that each image of the visual targets undergoes due to the combination of rotations applied by the reflections on the optical elements of the optical systems of the accessory device. This will be described with more detail hereafter in reference to figures 13 to 17.

The accessory device 2 is placed between the exit apertures of the optical refraction elements 11, 12 of the refraction test unit 10 of the optometry device 1 and the eye of the subject. The presence of the accessory device 2 therefore modifies the physical and/or optical distance between the eye and the corresponding optical component 11A, 12A of the refraction test unit. This physical and/or optical distance typically increases. Consequently, the parameter representative of the relative position of the eye of the subject and the corresponding optical component 11A, 12A of the optical refraction element 11, 12 of the optometry device 1 presents a final value determined in presence of the accessory device different from the reference value, in particular higher than the reference value.

Figures 2, 4 and 8 show the optical vertex distance d2, d3 in the presence of the accessory device with the first mirror in the active position.

Figures 3 and 7 shown the optical vertex distance d1 in the presence of the accessory device with the first mirror in the retracted position.

The size and the orientation of the optical elements of each optical system 41, 42 of the accessory device 2 may be adjusted to minimize the optical distance between the eye of the subject and the optical component 11A, 12A of the refraction test unit 10 of the optometry device 1 when the accessory device 2 is mounted on said optometry device 1.

In the example shown on figures 2 to 4, the size and the orientation of the two mirrors used as optical elements 411, 412, 421, 422 may be adjusted to minimize this optical distance.

In practice, the final value of the optical distance between the optical component 11A; 12A of the optometry device 1 and the eye of the subject is for example comprised between 20 and 80 mm, preferentially about 50 mm.

Moreover, the presence of the accessory device 2 may modify the relative position and/or orientation of the eye E1, E2 of the subject and the image of the visual target seen by the eye of the subject.

The accessory device 2 may be attached on the optometry device 1by any suitable mean known from the man skilled in the art, for example screwed, glued, or attached by magnets or any other mechanical means on the housing of the optometry device.

The accessory device 2 may be removable from the optometry device 1 or fixed. In the case where it is removable, easily removable mounting means may be used such as snap-fitting means.

Two embodiments of the accessory device 2 are represented on figures 5 to 8 and 9, respectively. As shown on figures 5 to 9, in each embodiment the optical part 40 of the accessory device 2 comprises a housing 43 which is configured to be attached to the refraction test unit 10 of the optometry device 1.

Each optical system is received in said housing 43.

In practice, the housing 43 is attached on the optometry device 1. It is preferably attached in a fixed position, non-movable on the optometry device 1.

The housing 43 comprises here two frames 43A, 43B with a substantially rectangular outline (figures 6 and 9). Each frame 43A, 43B is attached to one of the refraction test elements 11, 12 (figures 5 and 9).

In the first and second embodiments of figures 5 to 8 and 9, each frame 43A, 43B comprises an end wall 44 configured to be place against the optical refraction element 11, 12 of the optometry device. It comprises a hole 44A, 44B allowing light rays to enter the accessory device 2. A lateral wall 45 frames this end wall on three sides.

The lateral wall 45 of each frame 43A, 43B comprises two parallel side walls 451 extending along a longitudinal axis L1 of the accessory device. The longitudinal axis is configured to be vertical when the accessory device 2 is in use.

The longitudinal axis L1 of the accessory device is configured to extend parallel to the rotation axis V1, V2 of the optical refraction elements 11, 12 of the optometry device 1.

The two frames 43A, 43B are united by a horizontal bar extending perpendicularly to the side walls.

The side walls 451 of each frame 43A, 43B are linked by a bottom wall 452 which extends perpendicular to the side walls 451.

An inside face of the bottom wall 452 of each frame 43A, 43B is inclined towards the subject about a transverse axis perpendicular to the longitudinal axis of the accessory device. This inside face supports a reflective surface forming the second optical element 412, 422 of each optical system.

In both embodiments, a flap 46 extends from one frame to the other. It is mounted on the lateral walls of the frame, on a rotation axis Y (figures 6 and 9). The rotation axis Y is substantially horizontal. It is parallel to the two reflective surfaces 411, 412, 421, 422 of each optical system.

The flap 46 comprises two coplanar elements of flap, each one mounted inside one of the two frames 43A. They are linked by a bridge 46B comprising in its front edge a recess 46A. The recess 46A is provided for the nose of the subject to extend between the two frames (figures 6 and 9).

A face of the flap 46 oriented towards the bottom wall 452 comprises a single reflective surface extending across the flap, or two separated reflective surfaces placed in correspondence to the bottom walls 452, forming the first optical elements 411, 421.

When the bridge 46B of the flap 46 lies against the side walls of the frame 43A, 43B, the first optical elements 411, 421 are in the active position (figures 5, 6 and 9).

To place them in the retracted position, the flap 46 is pivoted upwards about the rotation axis Y and blocked in this pivoted position (figure 7).

This example of implementation corresponds to the case represented on figure 11 where the reflective surfaces of the first optical elements of the optical systems are coplanar as well as the reflective surface of the second optical elements.

The accessory device 2 also comprises two arms 47 presenting an L shape for attaching the housing 43 to the optometry device 1. The arms 47 extend in a common mean plane.

Each frame 43A, 43B is linked to one of the arms 47.

Each arm 47 comprises a longitudinal part extending along the longitudinal axis L1 of the accessory device 2 and a transverse end part. The transverse end parts of the arms 47 extend away from each other. Each of the transverse end part comprises here a plate 48 whose shape correspond to the outline of a corresponding part of the support 13 of the optical refraction elements 11, 12 of the optometry device 1. Each plate 48 comprises magnets configured to interact with said corresponding part of the support 13 of the optical refraction elements 11, 12 of the optometry device 1 to attach the accessory device on the optometry device. In practice, a cover panel of the support 13 is removed and replaced with the plate 48 to attach the accessory device.

In the second embodiment of the accessory device shown on figure 9, the accessory device 2 moreover comprises image capture means 50 for capturing profile images of the eye of the subject.

The image capture means 50 comprise a sensor 56 such as cameras, each configured to capture profile images of one of the eyes of the subject. Each sensor 56 is part of a printed circuit board 55 powered through electric conductors 57. Each printed circuit board 55 is supported by a base 51. The base 51 comprise a first branch extending from the side walls of the frames 43A, 43B of the housing 43 of the optical part 40 of the accessory device 2, parallel to the common mean plane of the arms 47. The base 51 also comprise a second branch 52 extending perpendicularly to the first branch 51. A free end of the second branch 52 forms a reception plate 54 receiving the printed circuit board 55. The two printed circuit board thus extend in parallel to each other, facing one another.

Advantageously, the accessory device 2 also comprise a determination tool 31 configured to determine a vision correction power for the eye of the subject, taking into account said refraction powers provided by the refraction test unit 10 during said subjective refraction test and a final value of the parameter representative of the relative position of the eye and the optical component 11A, 12A of said optometry device 1 in the presence of said accessory device 2.

The determination tool provides a vision correction power of the subject's eyes taking into account the new optical path of light through the accessory device. An accurate value of the vision correction power may then be determined by the system comprising the optometry device and the accessory device.

In practice, the determination tool 31 comprises for example a software part programmed to calculate said vision correction power depending on said final value of said parameter representative of the relative position of the eye of the subject and the optical component 11A, 12A of the optometry device 1, determined in the presence of said accessory device 2.

It may be configured to be implemented by said control device 30 of the optometry device. The determination tool 31 is represented on the figures 2 and 3 as part of the control device 30.

The determination tool may be a software that is uploaded in the control device. In that case, the determination tool can calculate the vision correction power based on the refraction measurements made in the presence of the accessory.

The determination tool may also calculate the vision correction power based on the reference vision correction power and the final value of said parameter representative of the relative position of the eye of the subject and the optical component 11A, 12A of the optometry device 1. The determination tool may also take into account the reference value of said parameter representative of the relative position of the eye of the subject and the optical component 11A, 12A of the optometry device 1.

The accessory device 2 may also comprise an adjustment tool 32 to adjust a position and/or orientation of each visual target T1, T2 displayed by said display unit 20 based on the inclination of said inclined observation direction OBS2, OBS3 and/or to adjust the refraction powers of the optical component 11A, 12A of the optometry device 1 based on a value of a parameter representative of the relative position of the image of the visual target T1, T2 unit and the eye E1, E2 of the subject.

The adjustment tool 32 may comprise a software that may also be implemented by the control device 30 of the optometry device 1.

In a variant, said determination tool and/or adjustment tool may be implemented by one or more processors different from the processors of the control device of the optometry device.

The vision correction power VCP determined by the determination tool 31 takes into account the refraction power of the optical component 11A, 12A of the optometry device 1 used during the subjective test and the final value of the parameter representative of the relative distance between the eye and the optical component when using the optometry system 3 comprising the accessory device 2 for a subjective test in far vision conditions (figure 3) or near or intermediate vision conditions (figures 2 and 4).

In an embodiment, the determination tool is programmed to determine the reference vision correction power VCP(d0) for the reference value d0 of the vertex distance based on a refraction power of SPH(di) provided by an optical component 11A, 12A when placed at a working optical vertex distance equal to di with the following basic formula:

$$VCP(d0) = \cfrac{1}{\left[\cfrac{1}{SPH(di) - Pobj} + \cfrac{d0 - di}{1000}\right]} + Pobj$$

Where

- the working optical vertex distance di is in millimeters and corresponds here to the final value of the parameter representative of the relative position between the eye and the optical component of the optometry device; in the examples represented on the figures, di may be equal to d1, d2 or d3;
- Pobj is in diopter and is the inverse of the physical distance between the apex of the face of the optical component 11A at its virtual position VL oriented towards the eye of the subject and the image I1 of the visual target T1 seen by the eye through the optical component of the optometry, for example equal to 2.5D at 40 cm,
- SPH(di), in diopter, is the refraction spherical power of the optical component when the eye of the subject is placed at the working optical vertex distance di during the subjective test, the refraction power of the optical component being calibrated for an object at infinity, and
- d0 is the reference value of the optical vertex distance corresponding to a recommended optical vertex distance in the absence of the accessory, in millimeters.

The reference value of the optical vertex distance is usually equal to 12 mm and is similar to the average distance between the eye and the ophthalmic lens worn by the subject.

The working optical vertex distance di is equal in the example shown on the figures to d1 when the first mirror is in the retracted position (figure 3), and equal to d2 or d3 when the first mirror is in the active position (figures 2 and 4).

In the retracted and active position of the first mirror, the optical vertex distance d1, d2, d3 may be measured, for example deduced from a profile image of the eye looking through the optical system 3, the internal geometry of the system in each of the retracted and active position of the first mirror being predetermined, fixed, and known.

In short, for a myopic eye, the optical component 11A, 12A of the optometry device 1 comprises a divergent lens. If the divergent lens of the optometry device 1 is distant from the eye E1, E2 of the subject by a working optical vertex distance di longer than the reference value d0 of the optical vertex distance s, the divergent lens providing an adequate visual correction at the working optical vertex di and determined by the subjective refraction test exhibits a reduced focal distance as compared with the focal distance of the divergent lens providing an adequate visual correction at the reference value of the optical vertex distance.

The absolute power value of the divergent lens providing an adequate visual correction at working optical vertex distance di is then higher than that of the divergent lens providing an adequate visual correction at the reference value of the optical vertex distance.

For an hypermetropic eye, the optical component of the optometry device comprises a convergent lens. If the convergent lens of the optometry device is distant from the eye of the subject by a working optical vertex distance di longer than the reference value of the optical vertex distance, for example equal to 12 millimeters, the convergent lens providing an adequate visual correction at the working optical vertex distance di exhibits a longer focal distance as compared with the focal distance of the convergent lens providing adequate visual correction at the reference value of the optical vertex distance. The absolute power value of the convergent lens providing an adequate visual correction at the working optical vertex distance di is then lower than that of the convergent lens providing an adequate visual correction at the reference value of the optical vertex distance.

The reference vision correction power for the eye of the subject corresponds to the power of the divergent and/or convergent lens used in the optometry device without accessory device, as determined through the subjective refraction test with the reference value d0 of the optical vertex distance.

In case of astigmatism, the above basic formula is applied for a first value of power P1 equal to the sphere power SPH and for a second value of power P2 equal to the sphere power SPH plus the cylindrical power CYL: P2=SPH+CYL.

This formula is correct only for an object at infinity since optometry devices are using optical components with power calibrated at infinity, corresponding to Pobj=0 D in the formula above. At any other distance, this formula is exact only in the thin lens approximation.

The optometry device 1 is programmed, in the absence of the accessory device, to provide a reference vision correction power corresponding to the observation of a visual target placed at infinity from the eye of the subject.

The refraction power provided or displayed by the optometry device 1 without the accessory device 2 thus usually corresponds to a back vertex power calculated for a visual target at infinity.

In an embodiment of the accessory of the invention, said determination tool 31 is configured to determine said vision correction power depending on a value of a parameter representative of the relative position of the image of the visual target and the eye of the subject. It is also advantageously configured to take into account the accurate geometry and/or optical features of the optical component 11A; 12A of the optometry device 1.

Indeed, more accurate calculation can be made by ray tracing and the exact description of the geometry of the optical system, as described hereafter. in an example of implementation of the invention in a subjective test performed in near vision conditions with the optometry system of the invention.

The optical vertex distance between the eye and the optical component 11A, 12A of the optometry system 3 is equal to d2 (figure 2) or d3 (figure 4) and takes into account the optical path of light through the accessory device 2. The distance between the rear surface of the optical component 11A placed at the virtual position VL and the virtual image of the visual target is usually 40 cm. The refraction power provided by the optometry device correspond to a back vertex power calibrated for a visual target at infinity.

Said determination tool is then programmed to simulate the optical component 11A, 12A of the optometry device 1 that provides said refraction power, to use a light ray tracing method to calculate the image of the virtual visual target through the optical component 11A, 12A and to calculate the reference vision correction power from the value of the parameter representative of the relative position of the eye of the subject and the optical component 11A, 12A of the optometry device, here the optical vertex distance d2, d3 and the reference value of the optical vertex distance d0.

To simplify ray tracing calculations, the cornea apex and the eye rotation center of the eye E1 are virtually positioned on the reference observation direction OBS1 of the optical component 11A of the optometry device 1 at the optical vertex distance d2 from the rear surface of the optical component 11A, and an object point is placed on the reference observation direction OBS1 at a distance from the rear surface of the optical component 11A equal to the distance between the rear surface of the virtual position VL of the optical component 11A oriented towards the eye E1 and the center of the virtual image I1 of the visual target T1 seen through the accessory device 2.

The elements of the simulation are schematically shown on figure 18. The virtual position of eye E1 is defined by the position of the apex of its cornea C1 and its center of rotation CRO1.

Point J and J' represent two distinct positions of the apex of the rear surface of the optical component 11A oriented towards the eye E1. Point J represents a real position of the apex of the rear surface of the optical component 11A oriented towards the eye E1 when the optical vertex distance is equal to d2, d3, whereas point J' represents a theoretical position of the apex of the rear surface of the optical component 11A oriented towards the eye E1 when the optical vertex distance is equal to the reference value of the optical vertex distance. The optical vertex distance is defined here as the optical distance between the apex of the cornea C1 of the eye E1 and the apex of the rear surface of the optical component 11A of the optometry device, noted J or J'.

The real optical vertex distance d2, d3 between the eye E1 and the optical component 11A of the optometry device is longer than the reference value.

Points M and M' on the reference observation direction OBS1 represent the two corresponding real and theoretical object points. The distances MJ, M'J' are fixed and remain equal to the distance between the rear surface of the optical component 11A at the virtual position VL oriented towards the eye E1 and the virtual image I1 of the visual target T1 seen through the accessory device in figure 4.

The refraction power from the optometry device 1 obtained at the end of the refraction test is used to deduce the features of an optical model corresponding to the optical component 11A or the features of the optometry device 1 used without the accessory device 2. If the optical component 11A contains a continuously deformable lens, one can optimize its shape considering the refraction power provided by the optometry device 1 and a visual target placed at infinity.

The sagittal $S_F$ and tangential $T_F$ focus points corresponding to point M are calculated by ray tracing using an optical model reflecting the features of the optical component 11A deduced from the refraction power of the optometry device 1 obtained at the end of the refraction test.

The determination tool then calculates: the distances $JS_F$, $JT_F$ between the sagittal $S_F$ and tangential $T_F$ focus points and the real position J of the optical component 11A.

The reference vision correction power (VCP(d0)) corresponding to the reference value d0 of the optical vertex distance is then calculated based on the distance M'J' between the object point and the rear surface of the optical component of the optometry device in their theoretical position M', J', with M'J' = MJ, and the distances $J'S_F$, $J'T_F$ between the optical component of the optometry device in its theoretical position and the sagittal and tangential focus.

For instance, the spherical equivalent reference vision correction power VCP and astigmatism VCAST are calculated with the formula:

$$VCP(d0) = 1/M'J' + 0.5*(1/J'S_F + 1/J'T_F)$$

$$VCAST(d0) = 1/J'S_F - 1/J'T_F$$

with

$$J'S_F = J'S_F + di - d0$$

$$J'T_F = J'T_F + di - d0$$

where di = d2 or d3.

This formula may be used to determine the vision correction power, said vision correction power being a sphere, a spherical equivalent, a cylinder power, or an astigmatism.

In the case where a virtual image of the visual target is considered, the distances MJ, M'J', $J'S_F$ and $J'T_F$ above mentioned are algebraic values with signs.

In an embodiment, the distance between the eye E1, E2 of the subject and the optical component 11A, 12A of the optometry device 1, in particular, the optical vertex distance, is measured using image capture means such as the ones described in reference to figure 9 above. The camera is positioned such that it is configured to capture images showing the eyes of the subject. It also comprises means to determine the distance between the camera and the optical component of the optometry device.

Alternatively, the camera is positioned such that it is configured to capture images showing the eyes of the subject and the optical component of the optometry device.

In another variant, said camera comprises a Time-Of-Flight camera.

Alternatively, the distance between the eye of the subject and the optical system of the accessory device 2 is calculated by addition of the total optical path length of the accessory device and the distance between the accessory device and the optometry device.

In another example of implementation of the invention, said determination tool may comprise a database comprising data relative to said vision correction power for the eye of the subject linked to values of said parameter representative of the relative position of the eye of the subject and the optical component of the optometry device, determined in the presence of said accessory device. The database may also comprise data relative to said vision correction power for the eye of the subject linked to the refraction power of the optical component of the optometry device, in particular the refraction power obtained in the last step of the subjective test corresponding to an optimum for visual correction of the visual defect of the eye of the subject.

The database may comprise a table or a graph. In that case, the vision correction power adapted to the subject's eye can be read on the database considering these data. Examples of such databases are given in the following tables 1 to 3.

The vision correction power corrected to be adapted to said target distance between eye and ophthalmic lens of 12 millimeters based on the value of the spherical refraction power (Rxsph) of the optical component 11A, 12A obtained in the last step of the subjective test in near vision conditions and the real optical vertex distance between the cornea of the eye and the optical component (d2) of the optometry device is shown in the table 1 below for an image of the visual target located at 40 cm from the optical component rear surface.

Table 1

| Rxsph | d2 (mm) | | | | | |
|---|---|---|---|---|---|---|
| | 7 | 9 | 12 | 15 | 17 | 20 |
| 10 | 9.53 | 9.62 | 9.76 | 9.90 | 10.00 | 10.16 |
| 9 | 8.62 | 8.68 | 8.78 | 8.88 | 8.95 | 9.06 |
| 8 | 7.70 | 7.74 | 7.80 | 7.87 | 7.91 | 7.99 |
| 7 | 6.76 | 6.79 | 6.82 | 6.86 | 6.89 | 6.93 |
| 6 | 5.82 | 5.83 | 5.85 | 5.86 | 5.87 | 5.89 |
| 5 | 4.87 | 4.87 | 4.87 | 4.87 | 4.87 | 4.86 |
| 4 | 3.91 | 3.91 | 3.89 | 3.88 | 3.87 | 3.86 |
| 3 | 2.95 | 2.93 | 2.92 | 2.90 | 2.89 | 2.87 |
| 2 | 1.97 | 1.96 | 1.94 | 1.92 | 1.91 | 1.89 |
| 1 | 0.99 | 0.98 | 0.97 | 0.95 | 0.95 | 0.94 |
| 0 | -0.01 | -0.01 | -0.01 | -0.01 | -0.01 | -0.01 |
| -1- | -1.01 | -1.00 | -0.98 | -0.96 | -0.95 | -0.93 |
| -2 | -2.02 | -1.99 | -1.95 | -1.91 | -1.88 | -1.85 |
| -3 | -3.04 | -2.99 | -2.92 | -2.85 | -2.81 | -2.74 |
| -4 | -4.07 | -3.99 | -3.89 | -3.79 | -3.72 | -3.63 |
| -5 | -5.10 | -5.00 | -4.85 | -4.71 | -4.62 | -4.49 |
| -6 | -6.15 | -6.01 | -5.82 | -5.63 | -5.52 | -5.35 |
| -7 | -7.20 | -7.03 | -6.78 | -6.55 | -6.40 | -6.19 |
| -8 | -8.26 | -8.05 | -7.74 | -7.46 | -7.27 | -7.02 |
| -9 | -9.33 | -9.07 | -8.70 | -8.35 | -8.14 | -7.83 |
| -10 | -10.41 | -10.10 | -9.66 | -9.25 | -8.99 | -8.63 |

The difference between the vision correction power thus determined and the vision correction power calculated with no correction by the optometry device 1 of the state of the art is shown is the table 2 below.

Table 2

| Rxsph | d2 (mm) | | | | | |
|---|---|---|---|---|---|---|
| | 7 | 9 | 12 | 15 | 17 | 20 |
| 10 | -0.47 | -0.38 | -0.24 | -0.1 | 0 | 0.16 |
| 9 | -0.38 | -0.32 | -0.22 | -0.12 | -0.05 | 0.06 |
| 8 | -0.3 | -0.26 | -0.2 | -0.13 | -0.09 | -0.01 |

(continued)

| Rxsph | d2 (mm) | | | | | |
|---|---|---|---|---|---|---|
| | 7 | 9 | 12 | 15 | 17 | 20 |
| 7 | -0.24 | -0.21 | -0.18 | -0.14 | -0.11 | -0.07 |
| 6 | -0.18 | -0.17 | -0.15 | -0.14 | -0.13 | -0.11 |
| 5 | -0.13 | -0.13 | -0.13 | -0.13 | -0.13 | -0.14 |
| 4 | -0.09 | -0.09 | -0.11 | -0.12 | -0.13 | -0.14 |
| 3 | -0.05 | -0.07 | -0.08 | -0.1 | -0.11 | -0.13 |
| 2 | -0.03 | -0.04 | -0.06 | -0.08 | -0.09 | -0.11 |
| 1 | -0.01 | -0.02 | -0.03 | -0.05 | -0.05 | -0.06 |
| 0 | -0.01 | -0.01 | -0.01 | -0.01 | -0.01 | -0.01 |
| -1 | -0.01 | 0 | 0.02 | 0.04 | 0.05 | 0.07 |
| -2 | -0.02 | 0.01 | 0.05 | 0.09 | 0.12 | 0.15 |
| -3 | -0.04 | 0.01 | 0.08 | 0.15 | 0.19 | 0.26 |
| -4 | -0.07 | 0.01 | 0.11 | 0.21 | 0.28 | 0.37 |
| -5 | -0.1 | 0 | 0.15 | 0.29 | 0.38 | 0.51 |
| -6 | -0.15 | -0.01 | 0.18 | 0.37 | 0.48 | 0.65 |
| -7 | -0.2 | -0.03 | 0.22 | 0.45 | 0.6 | 0.81 |
| -8 | -0.26 | -0.05 | 0.26 | 0.54 | 0.73 | 0.98 |
| -9 | -0.33 | -0.07 | 0.3 | 0.65 | 0.86 | 1.17 |
| -10 | -0.41 | -0.1 | 0.34 | 0.75 | 1.01 | 1.37 |

Table 3 shows the estimation of the difference in astigmatism (Ast_diff) for an object located at 40 cm of the rear surface of the optical component 11A, 12A of the optometry device 1 and a distance between eye and ophthalmic lens of 12 mm, for different values of cylindrical refraction power (Rx_cyl) of the optical component 11A, 12A of the optometry device 1 obtained in the last step of the subjective test.

Table 3

| Rx_cyl (D) | Ast_diff (D) |
|---|---|
| 0 | 0.03 |
| 1 | -0.06 |
| 2 | -0.13 |
| 3 | -0.19 |
| 4 | -0.26 |

The presence of the accessory device 2 may also modify the relative position of the image of the visual target seen by the eye of the subject and the eye of the subject. In particular, the deviation introduced to provide a convergent observation implies that a virtual image I1, I2 of the visual target T1, T2 is perceived at near vision distance from the eye E1, E2 of the subject (figure 4).

To create also a need of accommodation in relation with the close position of the virtual image of the visual target, the refraction power of the optical component 11A, 12A of the optometry device 1 may be adjusted by a value Pscreen-Pobj, Pscreen being the actual proximity of the visual target as displayed (close to 0 or 0.2D for 5m distance...), Pobj being the proximity targeted for the virtual image of the visual target (2.5D i.e. 40cm for Near). The proximity is equal to the inverse of the distance.

The adjustment tool 32 of the accessory device 2 may then comprise a software part programmed to adjust the refraction power of the optical component 11A, 12A of the optometry device 1.

The adjustment tool 32 of the accessory device 2 may also comprise a software part programmed to adjust the position and/or orientation of each visual target T1, T2 displayed by said display unit 20 based on the angle of inclination of said inclined observation direction OBS2, OBS3, as described above.

This adjustment is determined to ensure that the image 11', 12' of the visual target T1, T2 as seen by the eye of the subject presents a predetermined position and/or orientation.

In particular, each set of first 411, 412 and second 421, 422 optical elements may be rotated about a rotation axis X1, X2 (figure 12). This rotation axis X1, X2 is configured to be parallel to the rotation axis V1, V2 of the optical refraction elements 11, 12 of the optometry device 1, when the accessory device 2 is attached to the optometry device 1.

As a consequence, when the optical refraction elements 11, 12 of the optometry device 1 are rotated about their rotation axis V1, V2, it is possible to rotate the corresponding set of first and second optical elements to keep an accurate alignment of all the optical components. This is especially the case when the accessory device is used to provide an inclined observation direction corresponding to a convergent gaze direction.

In the case where the position and/or orientation of said optical systems are adjusted independently from each other, as shown in figure 12, the position and the orientation of the visual targets displayed by the display unit 20 must be adapted to the orientation of the subject's gaze direction, as described hereafter. Indeed, if the first and second optical elements 411, 422, 421, 422 of both optical systems of both eyes are not in the same plane, the visual target seen by each eye of the subject will be rotated about the inclined observation direction. Preferably in this case, the adjustment tool 32 of the accessory device comprises a software part programmed to rotate symmetrically the visual target for each eye to compensate for the cyclorotation that each image undergoes due to the combination of rotation implied by the mirrors of each optical system 41, 42.

Figure 13 shows an example of visual targets T1, T2 as displayed by the display unit 20 of the optometry device without any adjustment.

Figure 14 represents schematically the two images I1, I2 of said visual targets T1, T2, as seen by the two eyes of the subject when looking through the accessory device 2 and optometry device 1 in the configuration of figure 13.

The two images I1, I2 of the visual targets T1, T2 may be produced based on a single image or two identical or different images. Each of them is guided to one of the eyes of the subject. Because of the rotation of the optical elements of the accessory device 2, the images I1 I2 of the visual targets T1, T2 are rotated. Figure 14 shows schematically the images I1, I2 of the visual targets as seen by each eye of the subject.

Figure 15 shows the superposition of the two images I1, I2 of the visual targets, as seen in binocular vision by the subject. The subject will not be able to see a clear image as represented in figure 15: the fusion of the two images I1, I2 of the visual targets T1, T2 cannot occur as they cannot be superimposed.

The position and orientation of the visual targets T1, T2 produced by the display unit 20 position and orientation should then be adapted in order that both images 11', 12' of the visual targets reflected by the accessory device 2 are accurately superimposed.

Figure 16 shows corrected visual target T1', T2' as displayed by the display device.

Each corrected visual target T1', T2' is oriented in such a way that the images 11', 12' of the corrected visual targets are accurately superposed by binocular vision of the subject as shown in figure 17.

In practice, the correction consists in a rotation of the visual target T1, T2 initially displayed for each eye by a correction angle value Acorr. The correction angle value Acorr is calculated based on the convergence angle A2 and the downward angle A1 values of the inclined observation direction obtained thanks to the accessory device 2, according to the following formula:

$$Acorr = A2 * \sin(A1).$$

The use of the accessory device 2 with the optometry device 1 allows moving the gaze direction of the eye of the subject downwards without changing the position of the optometry device 1, in particular of the refraction test unit 10.

This solution has many advantages:

- it is not necessary to modify the position or orientation of the optometry device nor the position and/or orientation of the head of the subject when switching from straight ahead gaze direction for far vision refraction test to downwards gaze direction for near vision refraction test with the optometry device 1,
- the accessory device is a simple and light add-on device,
- the accessory device may be configured to be used with any kind of optometry device,
- the use of the accessory device allows performing near vision test with comfortable ergonomics of the optometry device which does not lean on the cheekbone of the subject while performing near vision tests.

The accessory device 2 may also be used to create a convergence between the two eye gaze directions. This solution has the following advantages:

- there is no necessary adjustment of the optometry device, in particular of the relative position and/or orientation of the refraction test elements 11, 12 of the refraction unit 10 when switching from a far vision refraction test to a near vision refraction test.
- it avoids limitation for small pupillary distance in near vision conditions, as shown in figure 4,
- it allows to perform the subjective refraction test with the optometry device for any visual distance between far and near vision conditions with the same display device.

The optometry device described here is a conventional optometry device used in a subjective refraction test for determining the refractive features of the eye of the subject. The accessory device is then used for determining the refractive features of the eye of the subject.

Alternatively, the accessory device may be used with the same king of optometry device or any other compatible optometry device, for testing different features of a subject's eye such as visual performance, dominant eye, vergence, phoria, objective or subjective features of the subject's eye.

## Claims

1. Accessory device (2) for testing a subject's eye (E1, E2) in conditions of near or intermediate vision, designed to be used with an optometry device (1) for measuring a subjective value of refraction of the eye (E1, E2), said optometry device (1) comprising:

    - a refraction test unit (10) having an optical component (11A, 12A) for providing different refraction powers to the eye (E1, E2) of the subject during a subjective refraction test, and
    - a display unit (20) adapted to produce a visual target (T1, T2) for the subject's eye (E1, E2), an image of said visual target (T1, T2) being visible through said refraction test unit (10) along a reference observation direction (OBS1),
    said accessory device (2) comprising an optical part (40) configured to be mounted on the refraction test unit (10) of the optometry device (1), comprising an optical system (41, 42) configured to deviate the image of the visual target (T1, T2) from said reference observation direction (OBS1) towards an inclined observation direction (OBS2, OBS3) along which the image of the visual target (T1, T2) is visible through the refraction test unit (10) and the optical part (40) of the accessory device (2), said inclined observation direction (OBS2, OBS3) defining a non-zero angle (A1, A2) with said reference observation direction (OBS1) when the optical part (40) is mounted on said refraction test unit (10) of the optometry device (1) and corresponding to a downward gaze direction of the subject and/or to a convergent gaze direction of the subject.

2. Accessory device (2) according to claim 1, wherein said optical system (41, 42) comprises a reflective or semi-reflective surface and/or an optical lens.

3. Accessory device (2) according to any one of claims 1 and 2, wherein said optical system (41, 42) comprises two optical elements (411, 412, 421, 422), a first one (411, 421) of said two optical elements being configured to be placed at an active position in front of an exit aperture of said optometry device (1) to deflect said image of the visual target (T1, T2) and convey it to a second (412; 422) of said two optical elements, said second optical element (412; 422) being configured to deflect the image of the visual target along said inclined observation direction (OBS2).

4. Accessory device (2) according to claim 3, wherein said first optical element (411, 421) is movable between said active position where it is placed in front of the optical component (11A, 12A), on said reference observation direction (OBS1) of said optometry device (1) and a second position where it is placed outside of said reference observation direction (OBS1).

5. Accessory device (2) according to any one of claims 1 to 4, wherein said accessory device (2) comprises a determination tool (31) configured to determine a vision correction power for the eye (E1, E2) of the subject, taking into account a final value of a parameter representative of the relative position of the eye (E1, E2) and the optical component (11A, 12A) of said optometry device (1) in the presence of said accessory device (2).

6. Accessory device (2) according to claim 5, wherein said determination tool (31) is configured to determine said vision correction power depending on a value of a parameter representative of the relative position of the image of the visual target (T1, T2) and the eye (E1, E2) of the subject.

7. Accessory device according to any one of claims 1 to 6, wherein said determination tool (31) comprises a software part programmed to calculate said vision correction power.

8. Accessory device according to claim 7, wherein said optometry device (1) comprises a control device (30) programmed to determine a reference vision correction power for the eye (E1, E2) of the subject taking into account said refraction powers provided by the refraction test unit (1) to the eye (E1, E2) of the subject and a reference value of a parameter representative of a relative position of the eye (E1, E2) and the optical component (11A, 12A) in the absence of said accessory device (2), and said software part is configured to be implemented by said control device (30) of the optometry device (1).

9. Accessory device according to any one of the preceding claims, wherein said accessory device (2) comprises an adjustment tool (32) configured to adjust a position and/or orientation of each visual target displayed by said display unit based on the inclination of said inclined observation direction and/or to adjust the refraction powers of the optical component (11A, 12A) of the optometry device (1) based on a value of a parameter representative of the relative position of the image of the visual target (T1, T2) and the eye (E1, E2) of the subject.

10. Accessory device according to any one of claims 1 to 6, wherein said determination tool comprises a database comprising data relative to said vision correction power for the eye of the subject linked to values of said magnitude representative of the relative position of the eye of the subject and the optical component of the optometry device, determined in the presence of said accessory device.

11. Accessory device according to any one of claims 1 to 10, wherein said optical part (40) comprises a housing (43) where said optical system (41, 42) is housed, said housing (43) being configured to be attached to the refraction test unit (10) of the optometry device (1).

12. Accessory device according to claim 3, wherein said refraction test unit (10) of the optometry device (1) having two optical components (11A, 12A) for providing different refraction powers to both eyes (E1, E2) of the subject, said optical part (40) of the accessory device (2) includes two optical systems (41, 42), each optical system (41, 42) being configured to be placed in front of one of said two optical components (11A, 12A) of said optometry device (1), allowing testing the eyes (E1, E2) of the subject with binocular vision, each optical system (41, 42) comprising said first and second optical elements (411, 412, 421, 422), the two first optical elements (411, 421) extend in a same first plane and the two second optical elements (412, 422) extend in a same second plane, the relative positions and/or orientations of said two first optical elements (411, 421) and the relative positions and/or orientations of said two second optical elements (412, 422) being fixed.

13. Accessory device according to claim 3, wherein said refraction test unit (10) of the optometry device (1) having two optical components (11A, 12A) for providing different refraction powers to both eyes (E1, E2) of the subject, said optical part (40) of the accessory device (2) includes two optical systems (41, 42), each optical system (41, 42) being configured to be placed in front of one of said two optical components (11A, 12A) of said optometry device (1), allowing testing the eyes (E1, E2) of the subject with binocular vision, each optical system comprising said first and second optical elements (411, 412, 421, 422), the position and/or orientation of each first optical element (411, 421) of said optical systems are adjustable independently from one another and the position and/or orientation of each second optical element (412, 422) of said optical systems are adjustable independently from one another.

14. Optometry system comprising an accessory device (2) for testing a subject's eye in ergonomic conditions of near or intermediate vision according to any one of claims 1 to 13 and an optometry device (1) for measuring a subjective value of refraction of the eye, said optometry device (1) comprising:

   - a refraction test unit (10) having an optical component (11A, 12A) for providing different refraction powers to the eye (E1, E2) of the subject, and
   - a display unit (20) adapted to produce a visual target (T1, T2) for the subject's eye (E1, E2), an image of said visual target (T1, T2) being visible through said refraction test unit (10) of the optometry device (1), along a reference observation direction (OBS1),
   wherein said optical part (40) of the accessory device (2) is mounted on said refraction test unit (10).

15. Method for testing a subject's eye in conditions of far vision and near or intermediate vision with an optometry system according to claim 14, said method comprising:

- performing a visual test in far vision conditions while the subject observes the image of the visual target (T1, T2) displayed by the display unit (20) of the optometry device (1) along said reference observation direction (OBS1), non-deflected by the accessory device (2),
- using said accessory device (2) to deflect said image of the visual target (T1, T2) towards said inclined observation direction (OBS2, OBS3),
- performing a visual test in near or intermediate vision conditions while the subject observes the image of the visual target (T1, T2) displayed by the display unit (20) of the optometry device (1) and deflected by the accessory device (2), along said inclined observation direction (OBS2, OBS3), without moving the refraction test unit (10) between performing said visual test in far vision and performing said visual test in near or intermediate vision.

# Fig.1

# Fig.2

# Fig.3

# Fig.4

**Fig.5**

**Fig.6**

**Fig.7**

**Fig.8**

# Fig.9

# Fig.10

**Fig.11**

**Fig.12**

**Fig.18**

**Fig.13**

T1

HEV

T2

HEV

**Fig.14**

I1

HEV

I2

HEV

**Fig.15**

I1    I2

HEV

**Fig.16**

T1'

HEV

T2'

HEV

**Fig.17**

I2'    I1'

HEV

Europäisches
Patentamt

European
Patent Office

Office européen
des brevets

## EUROPEAN SEARCH REPORT

**Application Number**

EP 22 30 6676

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 082 422 A1 (ESSILOR INT [FR]) 2 November 2022 (2022-11-02) * paragraph [0011] – paragraph [0122] * ----- | 1,2,5,6, 8,10-15 | INV. A61B3/028 A61B3/032 |
| X | JP 2018 171140 A (NIDEK KK) 8 November 2018 (2018-11-08) * paragraph [0115] – paragraph [0116] * * paragraph [0120] * * paragraph [0150] – paragraph [0151] * ----- | 1,3,4,7, 9,14,15 | |
| X | JP 2014 147416 A (NIDEK KK) 21 August 2014 (2014-08-21) * paragraph [0028] – paragraph [0029] * * paragraph [0070] * ----- | 1,14,15 | |

**TECHNICAL FIELDS SEARCHED (IPC)**

A61B

The present search report has been drawn up for all claims

| Place of search | Date of completion of the search | Examiner |
|---|---|---|
| Munich | 3 April 2023 | Alvazzi Delfrate, S |

## ANNEX TO THE EUROPEAN SEARCH REPORT
## ON EUROPEAN PATENT APPLICATION NO.

EP 22 30 6676

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2023

| Patent document cited in search report | | Publication date | Patent family member(s) | | Publication date |
|---|---|---|---|---|---|
| EP 4082422 | A1 | 02-11-2022 | EP | 4082422 A1 | 02-11-2022 |
| | | | WO | 2022229033 A1 | 03-11-2022 |
| JP 2018171140 | A | 08-11-2018 | NONE | | |
| JP 2014147416 | A | 21-08-2014 | JP | 6205732 B2 | 04-10-2017 |
| | | | JP | 2014147416 A | 21-08-2014 |

For more details about this annex : see Official Journal of the European Patent Office, No. 12/82

EPO FORM P0459